Europäisches Patentamt

(19) European Patent Office

Office européen des brevets

(11) Publication number: **0 149 547**
**A2**

(12) # EUROPEAN PATENT APPLICATION

(21) Application number: **85300237.6**

(22) Date of filing: **14.01.85**

(51) Int. Cl.⁴: **C 12 Q 1/68**

(30) Priority: **18.01.84 US 572091**

(43) Date of publication of application:
**24.07.85 Bulletin 85/30**

(84) Designated Contracting States:
**AT BE CH DE FR GB IT LI LU NL SE**

(71) Applicant: THE BOARD OF TRUSTEES OF THE LELAND
STANFORD JUNIOR UNIVERSITY
Encina 105 Stanford University
Stanford, California 94305(US)

(72) Inventor: Sklar, Jeffrey L.
3934 Nelson Drive
Palo Alto California 94306(US)

(72) Inventor: Cleary, Michael L.
744 Coleman Avenue
Menlo Park California 94025(US)

(74) Representative: Harrison, David Christopher et al,
MEWBURN ELLIS & CO 2/3 Cursitor Street
London EC4A 1BQ(GB)

(54) Neoplastic diagnosis by detection of gene rearrangements.

(57) Polynucleotide hybridization is employed for detection
of neoplastic cells which can be characterized by gene
rearrangement. For example, B-lymphocyte neoplasias can
be determined from restriction fragments of DNA in the
immunoglobulin encoding locus.

This invention was developed in part under Grant No.
CA-34233 from the National Institute of Health. The U.S.
government may have rights in this invention.

EP 0 149 547 A2

./...

Croydon Printing Company Ltd.

FIG. IA

FIG. IB

## NEOPLASTIC DIAGNOSIS BY DETECTION
## OF GENE REARRANGEMENTS

The primary method of diagnosis of neoplasia is histologic evaluation of tissue biopsies. However, distinguishing between malignant and benign disorders in lymph nodes and other lymphoid tissues by light microscopy requires substantial expertise and is frequently problematical even for experts in a minor proportion but significant number of evaluations. Contributing to the problem is that antigenically stimulated lymphocytes may morphologically resemble neoplastic lymphocytes, while conversely, so-called well-differentiated neoplastic lymphocytes may be cytologically indistinguishable from normal unstimulated lymphocytes. Occasionally, poorly-differentiated metastatic carcinoma or melanoma may be mistaken for lymphoma or reactive conditions may coexist which obscure the malignancy.

It would therefore be of great advantage in neoplastic evaluations to have a method which provides certainty concerning the nature of the neoplastic tissue and permits ordinary technicians to evaluate biopsies with a high degree of certainty.

Analysis of immunologic markers is described by Levy et al., J. Exp. Med. (1977) 145:1014-1028 and Aisenberg et al., Am. J. Med. (1980) 68:206-213. Detecting uniform rearrangements in human B-cell leukemia is described by Korsmeyer et al., J. Clin. Invest. (1983) 71:301-313; Korsmeyer et al., Proc. Natl. Acad. Sci. USA (1981) 78:7096-7100 and Hieter et al., Nature (1981) 290:368-372. The DNA rearrangement for immunoglobulin production is described by Hozumi and Tonegawa, Proc.

Natl. Acad. Sci. USA (1976) 73:3628-3632 and Leder et al., (1980) in Immunology 80, Eds. Fougereau and Dausset (Academic Press, London) pp. 34-50. Rearrangements of the immunoglobulin locus in T-cells has been studied by Cory et al., Proc. Natl. Acad. Sci. USA (1980) 77:4943-4947 and Kurosawa, Nature (1981) 290:565-570. The determination of "null cell" lymphomas as B-cells has been studied by Warnke et al., N. Eng. J. Med. (1980) 303:293-300. The description of the genes in the lambda light chain locus has been described by Hieter et al., Nature (1981) 290:536-540.

According to the invention, the determination of the cellular origin in neoplasms is achieved by detecting the presence of rearrangements in mammalian chromosomal DNA at a locus subject to rearrangement upon differentiation. Particularly, probes are employed for determining the fragment size of restriction fragments containing at least a portion of a sequence present in the rearranged locus, for example, with B-cells, the constant regions of the light and/or heavy chains of immunoglobulins, and the fragment size compared to germline DNA. The presence of uniform, monoclonal rearrangements is diagnostic of a neoplastic state and in combination with morphological characteristics of the proliferative cells can be used in the diagnosis of lymphomas.

Methods and compositions are employed for the determination of neoplasia in lymphoproliferative disorders. The method involves detecting the presence of monoclonality of proliferating cells e.g. lymphocytes, which undergo a genomic rearrangement upon differentiation. For example, the method involves detecting the presence of DNA rearrangements at the immunoglobulin loci of mammalian chromosomal DNA. The rearrangements can be detected by cleaving chromosomal DNA at specific sites, and detecting changes in fragment size as a result of rearrangement of germline DNA to produce DNA sequences encoding for polypeptide chains, e.g. light and/or heavy immunoglobulin chains. Particularly, the chromosomal DNA may be restricted, the fragments separated by size and hybridized with a probe incorporating at least a portion of the rearranged sequences. For B-cells, this is the light and/or heavy constant regions of the immunoglobulins.

It should be appreciated, that there will be a background of rearranged fragments of various sizes based on normal differentiated lymphocytes, e.g. B-cells. However, where the lymphoproliferative disorder is based on a neoplasm, the neoplastic cells will be uniform and of one size, so that the fragments which are obtained from a lymphoma will be clearly distinguishable from the fragments obtained from normal cells. Thus, the subject method provides for detecting the presence of a B-cell lymphoma or other lymphoproliferative disorder which involves monoclonality.

The samples employed may be biopsy tissue, including lymph nodes or extranodal sites, e.g., spleen, or other tissue suspected of being neoplastic, e.g. bone marrow. The biopsy tissue may be stored in any convenient manner, being frozen, fixed, or the like. The biopsy tissue will normally be three-dimensional so as to include a substantial sampling of the cell population.

Preparation of DNA from the cell sample may be obtained in accordance with standard procedures. The tissue may be lysed by treatment with a proteinase, mechanically or chemically. The DNA is normally extracted employing phenol, and then freed of RNA.

The DNA is then examined for the presence of rearrangements in the rearranged chromosomal locus. That is, one wishes to determine the base pair separation between two restriction cleavage sites. With B-cells, at least one of the sites will usually be in or adjacent to the domain defining the constant region of either the heavy or light chain, for example the $C_\mu$ region of the heavy chain. This determination can be achieved employing probes which include at least in part sequences of loci encoding for at least a portion of a constant region, non-coding intron sequences or non-coding sequences flanking the constant region loci.

Various techniques can be employed which will detect the spacing between the two predetermined sequences involving polynucleotide probes. One method which is presently available and can be conveniently applied is to employ restriction enzymes to define the position of the second predetermined site with respect to the first predetermined site. Thus, for B-cells the recognition sequence will define the distance between the ends of a fragment including at least a portion of the constant region of the immunoglobulins. Enzymes may be employed having recognition sequences of four bases, six bases or more, preferably six bases or more. One or more restriction enzymes may be employed, usually from one to two restriction enzymes, so that the termini of the fragments may be the same or different. The DNA will be restricted to provide fragments which are primarily of from about 1 to 30kb, usually from about 5 to 25kb.

The probe will normally be at least about 100 bases, more usually at least about 200 bases, and will usually not exceed about 10 kilobases (kb), usually not exceeding about 8kb. The probe will desirably include a sequence which is substantially consumed and is in a region which has low allelic variability in the chromosome. For B-cells, the probes may include in-whole or in-part coding sequences for the constant region of the light chain, either lambda or kappa alleles, the constant region of the heavy chain or the joining region (J) or combinations thereof. Alternatively or in combination, usually in combination, non-coding regions will also be present in the probe. In addition, the probe for the constant heavy region may be $C_\mu$, that is the constant region of IgM. The probe for the heavy chain may encompass the J region and not extend significantly into the region encoding for the heavy constant region of IgM.

The probe for the mu region will generally range from about 0.5 to 5kb. The probe for the J region will generally range from about 0.5 to 10kb, more usually from about 1 to 8kb. The probes for the constant regions of the light chains will generally vary from about 1 to 5kb, more usually from about 1.5 to 4kb. For the kappa constant region a portion or all of the kappa constant region may be employed as the probe, the probe generally ranging from about 0.5 to 3.5kb, more usually from about 0.5 to 3kb. For the lambda locus, the probe may include one or more of the genes associated with the lambda locus. These genes relate to determinants of the lambda chain, which include Oz, Kern, Mcg, etc. That is, the probe will include the sequence encoding for the particular determinant site or an allele lacking the particular determinant site.

Probes may be used individually or in combination, where the fragment sizes between the rearranged DNA and the unrearranged (germline DNA) can be individually detected. The combinations may involve a single locus, e.g., the lambda locus or a combination of loci, e.g., the lambda locus and/or kappa locus and/or heavy constant region.

The genomic DNA may be fragmented employing restriction enzymes and then isolated. The various fragments may then be separated in a variety of ways to provide for sufficient discrimination of fragments differing by 500bp, preferably differing by not more than about 200bp. Conveniently, this can be achieved by employing gel electrophoresis in accordance with known techniques, e.g. agarose or polyacrylamide gels. The distance traveled by the various fragments will be related to their molecular weight, so that fragments containing the sequences complementary to the probes will be separated in accordance with their size. In addition, other DNA sequences of known size may be employed so as to provide internal standards for molecular weight determinations. The identity of the fragments may then be determined employing known techniques, for example, Southern hybridization with radioactive probes and detecting the fragments by autoradiography.

By knowing the size of fragments obtained with the restriction enzyme or combination of restriction enzymes for the unrearranged (germline) DNA, one can readily determine whether one or both of the alleles encoding for the locus of interest has undergone rearrangement. The detection of rearrangement is diagnostic of the neoplastic state of the disorder, and the probe DNA sequence will be diagnostic of the origin of the cells involved in the lymphoproliferative disorder.

The subject method is found to be extraordinarily sensitive, being able to detect less than 5%, more usually less than 3%, ranging from about 0.5 to 2.5% of lymphoma DNA compared to total DNA. Only small samples are required, usually about 1mg of tissue being sufficient for a determination as to a particular locus. Thus, where three determinations are required, usually 3mg or less of tissue will suffice. In addition, the subject method is able to detect rearrangements, where such rearrangements were not functional for producing a polypeptide, e.g. for B-cells, cytoplasmic immunoglobulin or surface immunoglobulin. Therefore, with the probes one can diagnose the origin of cells, where due to the absence of particular polypeptides, there would be no other indication of cellular origin.

Another application for the subject method is the screening of lymphoproliferative lesions in immunosuppressed patients, particularly where the immunosuppression has been associated with organ transplants. In those situations where immunological studies have failed to demonstrate surface or cytoplasmic immunoglobulin, the subject method can be employed to determine clonality and elucidate the biology of the disorder.

The following examples are offered by way of illustration and not be way of limitation.


EXPERIMENTAL

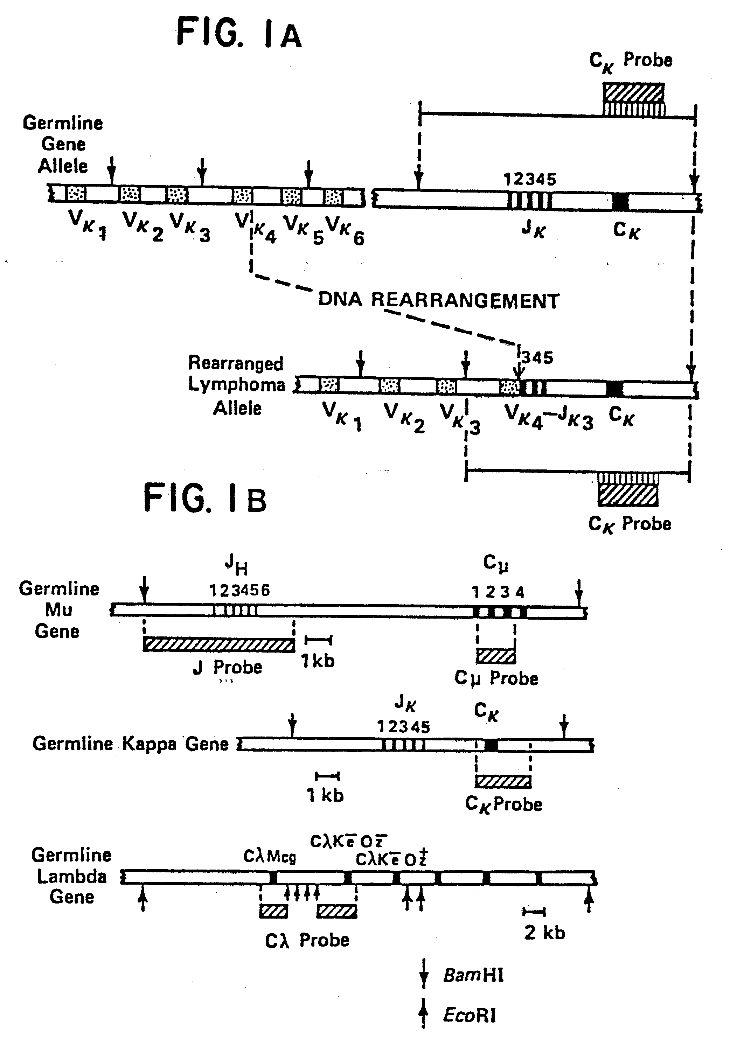

In Figure 1, Panel A schematically represents a hypothetical gene rearrangement for the light chain kappa locus. During B cell maturation prior to immunoglobulin production, one of the multiple kappa variable region genes ($V_{K4}$) undergoes somatic recombination with one of several separate joining region segments ($J_{K3}$) which lie directly upstream from a single kappa constant region gene ($C_K$). DNA rearrangement leaves the downstream BamHI restriction site (downward arrow to the right of $C_K$) unaltered while the BamHI site upstream and nearest to the constant region gene is changed in the rearranged lymphoma allele. This allows distinction between germline and rearranged kappa genes since the $C_K$ probe will detect different sized BamHI DNA fragments (bracketed lines at top and bottom of panel) using the Southern blot hybridization procedure. Panel B shows a chromosomal map of the germline configuration for the heavy chain, kappa and lambda constant region genes. The probes used in this work are indicated by crosshatched boxes. The heavy chain J region probe consisted of a 6.5 Kilobase (kb) BamHI-HindIII DNA fragment. A probe specific for the mu constant region gene consisted of a 1.4kb EcoRI fragment which includes the first, second and part of the third exons of the human $C_\mu$ gene. The J probe is preferable to the $C_\mu$ probe for demonstrating heavy chain rearrangements because the $C_\mu$ gene segment may be deleted during heavy chain class switching. The light chain kappa constant region probe

0149547

contained a 2.5kb <u>Eco</u>RI fragment spanning the entire human $C_K$ gene. The lambda constant region locus consists of a family of at least six closely linked, related genes, (e.g., Mcg, Ke⁻Oz⁻, and Ke⁻Oz⁺). (Hieter et al. (1981), <u>supra</u>). A combined $C_\lambda$ probe was used consisting of a 3.5kb <u>Eco</u>RI-<u>Hind</u>III fragment containing the Ke⁻Oz⁻$C_\lambda$ gene and a 2.5kb <u>Eco</u>RI-<u>Hind</u>III fragment containing the Mcg $C_\lambda$ gene.

<u>METHODS</u>

Biopsy tissues were routinely collected from the operating room, frozen in airtight plastic capsules by immersion in a dry ice-isopentane bath, and stored at -70°C for up to five years prior to use (Wood and Warnke, <u>J. Histochem and Cytochem</u> (1981), <u>29</u>:1196-1204). Normal, control tissues for two cases studied consisted of peripheral blood granulocytes and autopsy liver tissue.

DNA was extracted from lymph node biopsies and other tissues and purified according to standard procedures (Paterson and Sambrook, <u>J. Mol. Biol.</u> (1973) <u>73</u>:125-130). The starting material represented approximately 10-25mg (wet weight) of tissue.

After purification, high molecular weight DNA was digested with appropriate restriction enzymes according to conditions recommended by the supplier (BRL, Bethesda, Maryland). Digestion products were electrophoresed overnight in a 0.8% agarose gel (Robbins et al., <u>J. Biol. Chem.</u> (1979) <u>254</u>:6187-6195). After nicking of the DNA by ultraviolet light to reduce the average chain length, DNA fragments were transferred out of the agarose gels onto nitrocellulose filters, as described by Southern, <u>J. Mol. Biol.</u> (1975) <u>98</u>:503-517. The filters were then hybridized with radiolabeled pBR322 plasmid DNA carrying immunoglobulin DNA fragments. Hybridization reactions were carried out under conditions described by Alwin et al., <u>Proc. Natl. Acad. Sci. USA</u>

(1977) 12:5350-5354, using 50% formamide at 42°C. After extensive washing and drying of filters, autoradiography was performed at -70°C against a single intensifying screen for 12-72 hours.

Human genomic DNA fragments specific for the kappa (Hieter et al., Cell (1980) 22:197-207; Hieter et al., J. Biol. Chem. (1982) 257:1516-1522) and lambda (Hieter et al., Nature (1981) 294:536-540) constant regions and the heavy chain J and mu regions (Ravetch, Cell (1981) 27:583-591) were isolated from recombinant bacteriophage (provided by Dr. P. Leder, Harvard Medical School). These fragments were subcloned into the E. coli plasmid pBR322 using standard procedures. The positions of the DNA probe fragments with respect to the immunoglobulin genes are shown in Figure 1B. Plasmids containing immunoglobulin DNA were isolated from E. coli and nick-translated in vitro with $[^{32}P-\alpha]$dNTP deoxynucleoside triphosphates (Rigby et al., J. Mol. Biol. (1977) 113:237-251) to a specific activity of 3-5 x $10^8$cpm per microgram. Radiolabeled deoxynucleoside triphosphates were obtained from Amersham.

All lymphoma specimens were categorized histopathologically using the Working Formulation of Non-Hodgkin's Lymphomas ("The Non-Hodgkin's Lymphoma Pathologic Classification Project," Cancer (1982) 49:2112-2135). Analysis of immunologic surface and cytoplasmic markers was carried out in frozen sections as described by Wood and Warnke, supra.

## RESULTS

Immunoglobulin gene rearrangements are present in lymphomas but not in non-lymphoid control tissue from the same patient.

Analyses for heavy chain and kappa light chain gene arrangements using gel electrophoresis and Southern blotting (see Methods) were performed on DNA digested with the BamHI restriction enzyme; the lambda light chain locus was analyzed with DNA digested with the EcoRI restriction enzyme. Based on marker DNA fragments co-electrophoresed with DNA digests in these blots, the germline bands are of expected size: about 19.3kb (heavy chain); 12kb (kappa light chain); and 16, 14 and 8kb (lambda light chain).

Case 1 (Table 1) illustrates the data obtained from a lymph node biopsy diagnosed histologically as a diffuse, large cell lymphoma. The heavy chain J probe detected two bands in the lymphoma DNA, one of which comigrated with the single germline band obtained from peripheral granulocyte DNA. The second band, which migrated in a position below that of the germline band, represents clonal rearrangement of one heavy chain immunoglobulin allele. Separate blots prepared from the same DNA and hybridized with a probe for the kappa light chain gene also revealed a single clonally rearranged band which migrated slightly ahead of the position of the germline band, as well as a weaker band that comigrated with the germline band from granulocyte control DNA. Autoradiograms obtained from blots hybridized with the lambda light chain probe showed identical patterns for the control and lymphoma DNAs. The results obtained in this case are consistent with a clonal rearrangement of at least one allele of the heavy chain and kappa chain loci and correlate with immunologic phenotyping of frozen sections of this patient's lymphoma, which revealed mu and kappa surface immunoglobulin (Table 1).

A similar gel electrophoretic analysis was performed for Case 2, a diffuse, large cell lymphoma that developed in a patient with Wiskott-Aldrich syndrome. Non-lymphoma control DNA was extracted from autopsy liver, which was grossly and microscopically free of tumor. When the heavy chain J probe was used in the hybridization, three bands were seen, two of which migrated faster than the unrearranged germline band present in the control DNA. This finding can be explained by rearrangement of both heavy chain alleles in the lymphoma cells. The kappa light chain probe showed a single rearranged band representing a fragment larger than the germline fragment, in addition to a band which comigrated with the control DNA band. Hybridization of DNA from control and lymphoma tissues with the lambda light chain probe produced identical patterns. As with Case 1, these data are consistent with a clone of malignant cells which expressed mu-kappa immunoglobulin, in agreement with surface marker analyses, as shown in Table 1.

Varying amounts of germline band were present in analyses of both lymphoma specimens. These bands may have resulted from either an unrearranged immunoglobulin allele within tumor cells and/or from contamination of the tumor tissue with normal polyclonal lymphocytes, blood cells, fibrous tissue and blood vessels.

Immunoglobulin gene rearrangements are detectable in various histologic subtypes of B cell lymphoma.

Analyses for heavy chain gene rearrangements were performed as described previously using BamHI digested DNA extracted from lymph node biopsies. The case number coincides with the histologic diagnoses listed in Table 1. In most of the autoradiograms, there is a faint 12kb band of unknown origin which hybridizes weakly with the J-specific probe under the conditions used. This band is also detected in blots prepared from non-lymphoid DNA.

Results were obtained by gel electrophoresis from DNA isolated from lymph node biopsies representing a variety of conditions and the DNA probed with heavy chain J region DNA probes. Rearrangements were seen in all cases involving histologic subtypes of B cell lymphoma, as summarized in Table 1. Light chain rearrangements were also seen in all cases of B cell lymphoma. When present, the surface or cytoplasmic light chain identified in frozen sections corresponded to the light chain class that showed gene rearrangement (Table 1). Both kappa and lambda gene rearrangements were found in a single specimen containing only lambda surface immunoglobulin (Case 9), but no lambda gene rearrangement was found in lymphomas with kappa surface immunoglobulin. This observation conforms to the proposed hierarchy of immunoglobulin gene rearrangement, such that rearrangements of the lambda gene occur only after defective or non-productive rearrangements of both kappa gene alleles.

Immunoglobulin gene rearrangements are detectable in lymphomas lacking cellular immunoglobulin.

Two of the cases examined did not show staining for surface or cytoplasmic immunoglobulin but contained immunoglobulin gene rearrangements. Both Cases 10 and 4 (Table 1) are diffuse, large cell lymphomas which lack distinctive markers except for B1 (22) and Ia antigens. Despite the absence of immunoglobulin production in both cases, clonal heavy chain immunoglobulin gene rearrangements support the B cell lineage of these tumors. Moreover, light chain kappa gene rearrangements were found in each instance, while the lambda genes were in a germline configuration.

Immunoglobulin gene rearrangements are not seen in tissue specimens other than B cell lymphomas.

Four T cell lymphomas were examined, for example Case 11, a lymphoblastic lymphoma. All showed only germline bands and no detectable immunoglobulin gene rearrangements. Also examined was DNA from lymph nodes containing a wide variety of benign and reactive conditions, including reactive follicular hyperplasia (Case 3), angioimmunoblastic lymphadenopathy, rheumatoid arthritis, and sarcoidosis. Other non-B cell neoplastic processes were examined; these included acute myelocytic leukemia, acute monocytic leukemia, malignant histiocytosis, Hodgkin's disease, metastatic carcinomas and sarcomas, thymoma and Warthin's tumor of the salivary gland. DNAs from none of these specimens showed bands other than those comigrating with unrearranged germline bands.

High sensitivity of the Southern blotting technique for detecting clonal immunoglobulin gene rearrangements. To test the sensitivity of the Southern blotting technique for detecting minor subpopulations of malignant lymphocytes within biopsies, blots were prepared from known lymphoma DNA mixed with varying amounts of non-malignant lymph node DNA. After hybridizing with a kappa-specific probe, rearranged alleles could be confidently detected down to a level of 1-2.5 percent lymphoma relative to non-lymphoma DNA.

The next study investigated the monoclonality of B-lymphocyte proliferations in cardiac transplant recipients.

METHODS

The clinical features of the patients are summarized in Table 2. The ages of the patients ranged from 18 to 46 years with a mean of 35.8 years. Eight of the patients were male. The patients varied with respect to pre-existing cardiac disease prior to transplantation and immunosuppression: Four of ten patients presented with idiopathic cardiomyopathy, one presented with end-stage cardiomyopathy secondary to rheumatic heart disease, and five presented with atherosclerotic coronary artery disease. Seven of the patients received a single cardiac allotransplant and three patients received two or more cardiac allotransplants.

All patients were subjected to prolonged immunosuppression. For five patients the immunosuppressive regimen consisted of prednisone, azathioprine and antithymocyte globulin (ATG). Four patients received cyclosporin A and low dose prednisone in conjunction with ATG. One patient received cyclosporin A and low dose prednisone without ATG. The immunosuppressive regimens have been described in Jamieson et al., Suppression of immunity for cardiac transplantation, in Immunosuppressive Therapy, ed. J. R. Salaman, J.B. Lippincott Company, 1981; Oyer et al., Heart Trans. (1982) 1:285-290. The interval between initiation of immunosuppression and presentation with lymphoproliferative disorder varied widely, between 99 and 2575 days. However, patients receiving cyclosporin A developed lymphoproliferative disorders significantly sooner than patients receiving conventional immunosuppressive therapy (average interval of 132 days versus 1569 days).

Biopsy tissues were routinely collected from the operating room or at autopsy, frozen in airtight plastic capsules by immersion in a dry ice-isopentane bath, and stored at -70°C for up to five years prior to use (Wood and Warnke, Blood (1982) 59:913-922). Control tissues for two cases consisted of either peripheral blood granulocytes or grossly and microscopically normal splenic tissue removed at staging laparotomy.

Hemotoxylin and eosin-stained sections of formalin-fixed material obtained at surgery or postmortem examination were reviewed. The lesions were categorized histopathologically according to the Working Formulation of Non-Hodgkin's Lymphoma as recommended by an expert international panel.

Analysis of immunologic (surface and cytoplasmic) markers was carried out on frozen sections as described by Wood and Warnke, supra. Monoclonal antibodies against human heavy and light chain immunoglobulin were obtained from Becton-Dickinson, Inc. Goat antihuman $F(ab')_2$ antibody fragments reactive with light chains and heavy chains were obtained from Tago, Inc., Burlingame, CA. Anti-$B_1$ and anti-CALLA antibodies were obtained from Coulter Electronics, Inc., Hialeah, Florida. T015 antibody reactive against a B-cell antigen was provided by Harald Stein (Christian Albrecht University) and David Mason (Oxford University) (Stein et al., Clin Haematol (1982) 11:531-559).

Analyses for immunoglobulin gene rearrangements were performed as described in the previous study.

### RESULTS

Table 3 summarizes the histologic features of the lymphoproliferative disorders arising in the cardiac allotransplant recipients. All ten were histologically similar and were categorized as diffuse large cell lymphomas according to the recently proposed International Formulation for non-Hodgkin's lymphomas (histiocytic lymphomas in the Rappaport classification). In addition, eight of the cases showed features of a high grade immunoblastic lymphoma. Large foci of geographic necrosis were noted in all cases. Although there was frequent involvement of extranodal sites such as lung and soft tissues, the majority of these disorders either presented in or involved lymph nodes at some point during the course of the disease.

The results of immunological studies are summarized in Table 3. All ten cases were analyzed for immunoglobulins using monoclonal antibody reagents and/or polyvalent antibody fragments directed against each heavy chain class and both kappa and lambda light chains. Tissues examined consisted of lymph nodes, soft tissues, liver, brain and lung. All analyses for immunoglobulin or other antigens were performed on frozen sections of tissue obtained either at surgery prior to therapy for lymphoproliferative disease or, in one case, at autopsy 6 hours after death. Results of staining refer to histologically abnormal cells and not to apparently normal surrounding B cells, T cells or macrophages.

All ten cases stained uniformly for Ia-like antigen and nine stained for the B-cell antigens B1 and/or T015. In eight cases there was strong staining for all three of these antigens. Staining for CALLA, the common ALL-associated antigen, was negative in nine cases and equivocal in one. Nine of the ten cases showed no staining for multiple immunoglobulin heavy chain classes or either type of light chain. One case, Case 7, was positive for gamma-kappa immunoglobulin on all morphologically malignant cells but no cells expressed lambda light chains. In Case 8, the malignant cells in the lymph node biopsy did not stain for surface or cytoplasmic immunoglobulin. This same patient also underwent splenectomy for staging purposes and numerous nodules were present in the spleen. Sections of the nodules showed a minor population of malignant cells staining for mu-lambda immunoglobulin; however, the majority of the malignant cells did not stain for light or heavy chain, similar to the findings in the lymph node biopsy.

The following are the results obtained when tissue specimens from the ten transplant-associated lymphoproliferations were analyzed as described previously for heavy chain immunoglobulin gene rearrangements using a DNA probe specific for the joining region ($J_H$) of the heavy chain locus. Except for Case 7 which was restricted with EcoRI, the biopsy tissues were restricted with BamHI. In each lane, a band at 19kb represents the germline immunoglobulin locus. Any bands migrating in a position different from that of the 19kb band represent clonally rearranged heavy chain immunoglobulin alleles. In most lanes there is a weak 12kb band of unknown origin which cross-hybridizes with the J-specific probe under the hybridization conditions used. In several cases, the presence of two rearrangements is consistent with the interpretation that both heavy chain alleles have undergone rearrangement.

As summarized in Table 3, rearrangements were seen in all cases except Case 6 where only a germline band was observed. This case was analyzed using two different restriction enzymes (BamHI and EcoRI) since a rearranged band co-migrating with germline might not be evident with only one enzyme; however, no rearrangement was identified in digests performed with either enzyme. The presence of germline bands in the nine cases with rearrangements indicates that the tissue specimens contained unrearranged immunoglobulin alleles in addition to the rearranged allele(s). The germline alleles are contributed either by a single unrearranged allele in the tumor cells and/or by allelic loci in contaminating non-malignant cells, such as normal B cells, T cells, macrophages, vascular cells, or stromal cells present within the specimen.

Control tissues from two of the patients consisted of either peripheral blood cells obtained at the time of diagnosis (Case 7) or splenic tissue removed at staging laparotomy and uninvolved by disease (Case 8). In both cases, control tissue showed bands migrating only in a germline position. Inherited polymorphisms of the germline heavy chain gene analyzed with BamHI and EcoRI restriction enzymes appear to be very rare in the general population.

In order to guard against the unlikely possibility of interpreting heavy chain DNA polymorphisms as immunoglobulin gene rearrangements, DNAs from lymphoproliferative lesions for light chain immunoglobulin gene rearrangements were also analyzed. DNAs analyzed with a kappa light chain-specific probe after BamHI digestion (1, 3-7 and 9) revealed clonal rearrangement of kappa alleles in seven of the ten cases since bands were found migrating in a position other than that of the germline band at 12kb. In addition, three cases (2, 8 and 10) showed definite lambda light chain gene rearrangements, when DNA from these cases was analyzed with the combined $C_\lambda$ probe. The presence of two rearranged bands in several lanes is again consistent with rearrangement of both light chain gene alleles. As in autoradiograms analyzing heavy chain gene rearrangements, germline bands of variable intensity were seen in analysis of light chain genes in all specimens.

Cases exhibiting a clonal rearrangement of the kappa light chain gene included Case 6, which lacked a detectable heavy chain gene rearrangement. This finding violates the proposed hierarchy of immunoglobulin gene rearrangement in which heavy chain gene rearrangement precedes rearrangement of light chain genes.

It is evident from the above results, that the subject method provides a highly sensitive method for detecting monoclonal disorders, particularly B-cell lymphomas. In accordance with this method, rearrangement of the immunoglobulin DNA locus is employed as diagnostic of B-cell disorders. Furthermore, it is found that polymorphisms are either not frequently encountered or do not interfere with the subject method. In addition, the polyclonality of contaminating normal cells provides a background which does not interfere with the detection of a low percentage of monoclonality. Thus, by employing the subject method for determining the presence of monoclonality or rearrangement in the immunoglobulin locus, in conjunction with examination of the morphology of the cells, an accurate diagnosis of a neoplasia can be made.

Although the foregoing invention has been described in some detail by way of illustration and example for purposes of clarity of understanding, it will be obvious that certain changes and modifications may be practiced

Table 1:  Correlation of Immunoglobulin Gene Rearrangements
(Immunogenotype) with Immunoglobulin Antigens (Immuno-
phenotype) in Various Lymphoproliferative Conditions.

| Case | Histologic Diagnosis | Immuno-genotype | | | Immuno-phenotype | | |
|------|---------------------|-----|-----|-----|-----|-----|-----|
|      |                     | $\mu$* | κ | λ | $\mu$ | κ | λ |
| 1 | Diffuse large cell ML | R | R | G | + | + | - |
| 2 | Diffuse large cell ML | R | R | G | + | + | - |
| 3 | Reactive follicular hyperplasia | G | G | G | ND | ND | ND |
| 4 | Large cell immunoblastic ML | R | R | G | - | - | - |
| 5 | Diffuse small cleaved cell ML | R | R | G | + | + | - |
| 6 | Small non-cleaved cell ML, non-Burkitt's | R | R | G | + | + | - |
| 7 | Small lymphocytic ML | R | R | G | + | + | - |
| 8 | Unclassified ML | R | R | G | + | + | - |
| 9 | Lymphocytic ML, intermediate differentiation | R | R | R | + | - | + |
| 10 | Diffuse large cell ML | R | R | G | - | - | - |
| 11 | Lymphoblastic ML[+] | G | G | G | - | - | - |

ML = Malignant lymphoma; R = Rearranged immunoglobulin gene;
G = Germline; ND = Not determined

*Rearrangements of the heavy chain locus detected with heavy chain
J-specific probe were confirmed with the Cμ probe.  In each case
the Cμ probe hybridized to at least one rearranged band detected
with the J probe.

[+]T cell differentiation determined by analysis of surface markers
(Leu-1[+], Leu-2a[+], Leu-3a[+], Leu-4[+], Ia[-])

Table 2: Clinical Feature of Cardiac Allotransplants Recipients Who Developed Lymphoproliferative Disorders.

| Patient | Sex/ Age | Pre-existing cardiac disease | Number of transplants | Immuno-suppressive regimen | Interval to diagnosis of lymphoproliferation (days) | Status (Cause of death) |
|---|---|---|---|---|---|---|
| 1 | M/23 | CMP | 1 | Prednisone Azathioprine ATG | 1514 | Dead, 4 mo.[*+] (No autopsy; clinically CAD) |
| 2 | M/32 | CAD | 2 | Prednisone Azathioprine ATG | 2575/133 | Dead, 4 mo. (LD) |
| 3 | F/40 | CAD | 1 | Cyclosporin A Azathioprine ATG | 151 | Dead, 1 mo. (LD) |
| 4 | M/18 | CMP | 2 | Prednisone Azathioprine ATG | 938/782 | Dead, 10 mo. (LD) |
| 5 | M/39 | CMP/RHD | 1 | Cyclosporin A Prednisone ATG | 187 | Dead, 1 mo. (LD + infection) |
| 6 | F/43 | CMP | 1 | Prednisone Azathioprine ATG | 1487 | Dead, 3 mo. (LD) |

-------------------------------------------- C O N T I N U E D --------------------------------------------

0149547

Table 2: Clinical Feature of Cardiac Allotransplants Recipients Who Developed Lymphoproliferative Disorders. (CONTINUED)

| Patient | Sex/ Age | Pre-existing cardiac disease | Number of transplants | Immuno-suppressive regimen | Interval to diagnosis of lymphoproliferation (days) | Status (Cause of death) |
|---|---|---|---|---|---|---|
| 7 | M/44 | CMP | 1 | Cyclosporin A Prednisone | 122 | Alive, 6 mo.[+] |
| 8 | M/32 | CAD | 1 | Cyclosporin A Prednisone ATG | 99 | Dead, 3 mo. (LD + infection) |
| 9 | M/46 | CAD | 1 | Cyclosporin A Prednisone | 101 | Dead, 12 mo.[+] (CAD) |
| 10 | M/41 | CAD | 3 | Prednisone Azathioprine ATG | 1335/459/457 | Dead, 29 mo.[+] (No autopsy; cause unknown) |

CAD - atherosclerotic coronary artery disease

CMP - cardiomyopathy

RHD - rheumatic heart disease

ATG - antithymocyte globulin

LD - lymphoproliferative disorder

*Survival after diagnosis of lymphoproliferative disorder

[+]No apparent lymphoproliferative disease.

0149547

Table 3: Histologic Classification, Immunologic Phenotype and Immunoglobin Gene Rearrangements of Post-Transplant Lymphoproliferations.

| Patient | Histologic diagnosis | Site | Immunophenotype | | | | | | | | | |
|---|---|---|---|---|---|---|---|---|---|---|---|---|
| | | | $\kappa$ | $\lambda$ | $\mu$ | $\gamma$ | $\alpha$ | $\delta$ | Ia | $B_1$ | CALLA | T015 |
| 1 | LCI | Lung | - | - | - | - | - | - | + | -* | - | -* |
| 2 | LCI | Lung | - | - | - | - | - | - | + | + | - | + |
| 3 | LCI | Groin LN | - | - | - | - | - | - | + | + | - | -* |
| 4 | DLC | Thigh soft tissues | - | - | - | - | - | - | + | + | - | - |
| 5 | LCI | Auricular LN | -* | - | -* | - | - | - | + | + | - | + |
| 6 | LCI | Liver | - | - | - | - | - | - | + | + | - | + |
| 7 | LCI | Axillary LN | + | - | - | + | - | - | + | - | - | + |
| 8 | LCI | Axillary LN | - | - | - | - | - | - | + | + | ± | + |
| 9 | LCI | Inguinal LN | - | - | - | - | - | - | + | + | - | + |
| 10 | DLC | Brain | - | - | - | - | - | - | + | + | - | + |

-------------------------------- C O N T I N U E D --------------------------------------

```
*     = Minor population of staining cells        R    = Rearranged
LCI   = Large cell, immunoblastic lymphoma        G    = Germline
(G)   = Germline band with reduced intensity      DLC  = Diffuse large cell lymphoma
```

0149547

Table 3: Histologic Classification, Immunologic Phenotype and Immunoglobin Gene Rearrangements of Post-Transplant Lymphoproliferations. (CONTINUED)

| Patient | Histologic diagnosis | Site | Immunogenotype | | |
|---|---|---|---|---|---|
| | | | H | κ | λ |
| 1 | LCI | Lung | R | R | G |
| 2 | LCI | Lung | R | G | R |
| 3 | LCI | Groin LN | R | R | G |
| 4 | DLC | Thigh soft tissues | R | R | G |
| 5 | LCI | Auricular LN | R | R | G |
| 6 | LCI | Liver | G | R | G |
| 7 | LCI | Axillary LN | R | R | G |
| 8 | LCI | Axillary LN | R | (G) | R |
| 9 | LCI | Inguinal LN | R | R | G |
| 10 | DLC | Brain | R | G | R |

| * | = Minor population of staining cells | R | = Rearranged |
|---|---|---|---|
| LCI | = Large cell, immunoblastic lymphoma | G | = Germline |
| (G) | = Germline band with reduced intensity | DLC | = Diffuse large cell lymphoma |

0149547

CLAIMS :

1.      A method for detecting neoplasia in lymphoproliferative disorders involving monoclonality, which
comprises:

detecting the size of fragments of DNA from a
locus subject to rearrangement upon differentiation,
said DNA being obtained from a tissue sample suspected
of having said disorder, said fragments having predetermined terminal sequences, as compared to germline DNA,
by means of a probe which is complementary to a sequence
in said locus; and

comparing the fragment size to a fragment size
obtained from germline DNA having the same terminal
sequences.

2.      A method according to Claim 1, wherein said
tissue is lymph node tissue.

3.      A method according to Claim 1, where said
tissue is bone marrow.

4.      A method for detecting B-cell neoplasia in
lymphoproliferative disorders involving monoclonality,
which comprises:

detecting the size of fragments of DNA from
a locus subject to rearrangement upon differentiation,
said DNA being obtained from a tissue sample suspected
of having said disorder, said fragments having predetermined terminal sequences, as compared to germline DNA,
by means of a probe which is complementary to a sequence
in said locus; and

comparing the fragment size to a fragment size obtained from germline DNA having the same terminal sequences.

5.    A method according to Claim 4 wherein said probe is for a sequence homologous to the constant region of the heavy chain.

6.    A method according to Claim 5 wherein said sequence is in the J region.

7.    A method according to Claim 4 wherein said probe is for a sequence homologous to the constant region of the light chain.

8.    A method according to Claim 7 wherein said sequence is the J region.

9.    A kit for diagnosing neoplasia comprising probes of a size in the range of about 0.5 to 20kb having sequences homologous to at least one region in the immunoglobulin locus, said regions being the kappa, lambda and mu constant regions and J region.

10.    A kit according to Claim 9 wherein said regions are the kappa and lambda constant regions and J heavy region.

FIG. 1A

0149547

FIG. 1B